# EUROPÄISCHE PATENTANMELDUNG

(11) **EP 1 882 511 A2**
(43) Veröffentlichungstag der Anmeldung: **30.01.2008**
(21) Anmeldenummer: 07113043.9
(22) Anmeldetag: 24.07.2007
(51) Int. Cl.: B01D 46/10

(54) **Filtermedium mit bakterieller Wirkung**

(30) Priorität: 26.07.2006 DE 202006011647 U
(71) Anmelder: MANN+HUMMEL GmbH, 71638 Ludwigsburg (DE)
(72) Erfinder: Koch, Eugen, 75328 Schömberg (DE); Trautmann, Dr. Pius, 84130 Dingolfing (DE)

(57) **Zusammenfassung**

Es wird ein Filtermedium mit bakterizider Wirkung, insbesondere zur Filterung von Luft für den Innenraum von Kraftfahrzeugen, bereit gestellt, bestehend aus mindestens einer Filterschicht, in der partikuläre Verunreinigungen zurückhaltbar sind, und einer dieser Filterschicht nachgeschalteten bakteriziden Filterschicht. Die bakterizide Filterschicht ist auf der Reinluftseite der mindestens einen Filterschicht angebracht und durch eine Abstandsschicht von der mindestens einen Filterschicht beabstandet.

## Beschreibung

### Gebiet der Erfindung

Die vorliegende Erfindung bezieht sich allgemein auf ein Filtermedium. Insbesondere bezieht sich die Erfindung auf ein Filtermedium mit bakterieller Wirkung. Ganz speziell betrifft die Erfindung ein Filtermedium zur Filterung von Luft für den Innenraum eines Kraftfahrzeugs Des weiteren bezieht sich die vorliegende Erfindung auf ein Filtermodul.

### Hintergrund der Erfindung, Stand der Technik

Aus Filtermedien hergestellte Filterelemente dienen der Filterung von Fluidströmungen oder gasförmigen Medien, beispielsweise zur Filterung einer Luftströmung, die dem Fahrzeuginnenraum eines Kraftfahrzeugs zugeführt wird. Obwohl auf beliebige Filterelemente anwendbar, werden die vorliegende Erfindung sowie die ihr zugrunde liegende Problematik nachfolgend mit Bezug auf ein Filtermedium bzw. Filterelement zur Filterung von Luft für den Innenraum eines Kraftfahrzeugs beschrieben. Solche Filter werden nachfolgend auch kurz als Kfz-Innenraumluftfilter bezeichnet.

Die zunehmende Luftverunreinigung, insbesondere in Großstädten, in Verbindung mit dem Einsatz moderner Klimaanlagen macht es wünschenswert und auch erforderlich, die von außen in den Innenraum eines Kraftfahrzeuges geleitete und aufbereitete bzw. klimatisierte Luft mittels geeigneter Filter zu filtern. Hierfür kommen beispielsweise Partikelfilter oder Geruchsfilter oder alternativ auch kombinierte Partikel- und Geruchsfilter in Betracht, die in der Luft enthaltene Partikel und inhärente Gerüche aus der Umgebungsluft möglichst gut herausfiltern sollen. Solche Filter zur Filterung von Luft für den Innenraum eines Kraftfahrzeuges sind in einer Vielzahl von Ausführungsformen und Varianten allgemein bekannt, so dass auf deren Aufbau und Funktionsweise nachfolgend nur kurz eingegangen wird.

Da die Wirksamkeit von Filtern insbesondere von der Größe der von der Luft durchströmten Oberfläche des Filters abhängig ist, kommen für Kfz-Innenraumluftfilter überwiegend ziehharmonikaförmig oder zick-zack-förmig gefaltete Filtermedien, die häufig auch als plissierte Filtermedien bezeichnet werden, zum Einsatz. Durch die Faltung des verwendeten Filtermediums kann auf diese Weise abhängig von der Faltungshöhe und dem Faltungsabstand der verschiedenen Faltabschnitte des Filtermediums eine effektive Vergrößerung der von dem Luftstrom durchströmten Filterfläche ermöglicht werden.

Wie bereits erwähnt, gelangen bedingt die zunehmende Luftverschmutzung immer mehr Schadstoffe ins Innere eines Kraftfahrzeuges, so dass die Insassen einer zunehmenden Belastung ausgesetzt sind. Bei diesen Schadstoffen kann es sich bspw. um Endotoxine und Mycotoxine handeln, die Abbauprodukte der Zellwände von Pilzen und Bakterien darstellen, und von denen bekannt ist, dass sie Allergiestoffe für das menschliche Atmungssystem darstellen. Bei einigen Individuen können sie Asthmaanfälle auslösen und es ist nachgewiesen, dass sie eine immunologische Abwehrreaktion auslösen können. Ebenfalls bedenklich sind Pilzsporen, Bakteriensporen und Bakterien.

Die US 2003/0116022 A1 beschreibt einen Luftfilter für Klimaanlagen, wie sie in Büros, Wohnhäusern und Gebäuden für die medizinische Versorgung, wie zum Beispiel Krankenhäusern und Pflegeheimen verwendet werden. Der Filter umfasst eine Zusammensetzung, die ein Biozid enthält, das nicht an die Filteroberfläche gebunden, sondern so angepasst ist, dass es durch den sich auf dem Filter ansammelnden Feinstaub wandern kann, so dass die Schadstoffteilchen mit dem Biocid beschichtet werden. Geeignete Biocide sind zum Beispiel 2-Brom-2-nitropropane-1,3-diol, Isothiazolinverbindungen, Benzoesäure, Benzalkoniumhalogenide und dergleichen.

Aus der US 2006/0021302 A1 ist ein anti-mikrobieller Luftfilter bekannt, dessen Filtrationsmedium eine "prophylaktische" Verbindung aufweist, die dazu verwendet werden kann, die Anzahl von mikrobiellen Organismen zu verringern. Die "prophylaktische" Verbindung wird als separate Lage neben dem plissierten Filtermedium eingebracht. Bei der "prophylaktischen" Verbindung kann es sich um wasserlösliche Coenzyme, öllösliche Coenzyme, Pflanzenextrakte, Antibiotika, biozide Metalle, aliphatische und aromatische Fettsäuren und dergleichen handeln.

Schließlich offenbart die WO 2996/003515 A1 eine Luftbehandlungsvorrichtung für ein Teilchenfilter einer Klimaanlage eines Fahrzeugs. Die Vorrichtung besteht aus einem durchlässigen Container, der ein leicht flüchtiges Behandlungsmittel enthält. Der Container wird auf dem Teilchenfilter befestigt.

In Joerg C. Tiller et al., "Designing surfaces that kill bacteria on contact" wird die Fähigkeit von auf Glasplatten aufgebrachtem Poly(4-vinyl-N-hexylpyridiniumbromid) (Hexyl-PVP) beschrieben, verschiedene durch die Luft übertragene Bakterien bei Kontakt abzutöten. Filterelemente sind in dieser Druckschrift nicht angesprochen.

Die US 2005/0079379 A1 schließlich beschreibt ein Abdeckgewebe mit mindestens einer Stoffbahn, die einen elektrostatisch aufgeladenen Meltblown-Faservlies-Flor umfasst, der mit einer Fluorchemikalie mit einem schwachen kationischen Emulgator behandelt ist, um die Oberflächenenergie der Fasern zu reduzieren, um auf diese Weise ein Durchdringen und Benetzen durch ölige Nebel zu minimieren und damit die Wirkung der den Fasern aufgegebenen elektrostatischen Ladungen zu erhalten. Die Fasern können Polyvinyl-N-pyridiniumbromid enthalten. Das Gewebe wird für Gesichtsmasken und Schutzkleidung verwendet. Eine Verwendung für Innenraumfilter bei Kraftfahrzeugen ist nicht vorgesehen.

### Zusammenfassung der Erfindung

Es ist daher die Aufgabe der vorliegenden Erfindung, ein Filtermediun, insbesondere ein Filtermedium für Innenraumfilterelemente für Kraftfahrzeuge bereit zu stellen, das in der Lage ist, Mikroorganismen abzutöten und gleichzeitig einen Bewuchs des Filtermediums mit Bakterien, Pilzen und sonstigen Mikroorganismen und ein Durchwachsen wirkungsvoll zu verhindern.

Es ist eine weitere Aufgabe der vorliegenden Erfindung, solche Medien und damit Filterelemente zu liefern, die gleichzeitig eine Schonung der nutzenden Personen, also insbesondere der Insassen des Kraftfahrzeugs vor aus dem Filtermedium emittierten Stoffen bieten.

Diese und weitere Aufgaben werden durch das Filtermedium nach Anspruch 1 gelöst.

Weitere vorteilhafte Ausgestaltungen sind in den Unteransprüchen enthalten.

### Detaillierte Beschreibung der Ausführungsbeispiele

Das erfindungsgemäße Medium kann bspw. bei Innenraumfiltern für Kraftfahrzeuge zur Anwendung kommen. Weitere Einsatzbeispiele sind Staubsaugerfilter, Staubsaugerbeutel, Filterelemente für Gebäude- und Standklimaanlagen, Filterelemente für Luftreiniger, Atemfilter und dgl.

Im folgenden wird die Erfindung am Beispiel eines Innenraum-Luftfilterelements für ein Kraftfahrzeug beschrieben. Dem Fachmann ist jedoch klar, dass die Erfindung nicht darauf beschränkt ist, sondern, wie bereits oben erwähnt, auch anderweitig anwendbar ist.

Das erfindungsgemäße Filtermedium ist in der Lage, Mikroorganismen abzutöten und gleichzeitig einen Bewuchs des Filtermediums mit Bakterien, Pilzen und sonstigen Mikroorganismen und insbesondere ein Durchwachsen wirkungsvoll zu verhindern. Unter Durchwachsen versteht man die Ausbreitung von mycelbildenden Mikroorganismen durch eine Sperrschicht, z.B. eine biozide Schicht hindurch. Dies kann auch durch Bakterien geschehen, die sich durch diese Schicht hindurch ausbreiten.

Dazu wird erfindungsgemäß vorgeschlagen, ein Filtermedium mit bakteriziden Eigenschaften so zu gestalten, dass die Mikroorganismen abtötende Filterschicht auf der Reinluftseite der Filterschicht angebracht ist. Das Filtermedium besteht dabei aus mindestens einer Filterschicht, in der partikuläre Verunreinigungen zurückgehalten werden. Dieser Filterschicht nachgeschaltet ist die bakterizide Filterschicht, wobei diese bakterizide Filterschicht mit einer Abstandsschicht von der vorgeschalteten Filterschicht abgetrennt ist. Somit kann ein Durchwachsen auch von mycelbildenden Mikroorganismen wirkungsvoll verhindert werden. Die Abstandsschicht kann ebenfalls bakterizid ausgerüstet sein. Die Abstandsschicht kann dabei aus einem feinen Kunststoffgitter, einer Klebermatrix oder einem 3-D-Fasergestrick oder -gewirk bestehen. Bei Adsorptionsmedien, die z.B. mit Aktivkohle als Adsorbens ausgerüstet sind, kann die Adsorptionsschicht selbst als Abstandsschicht dienen. Bei Adsorptionsmedien dient die biozide Schicht gleichzeitig als Trägerlage für das Adsorbens.

Zur Beschichtung oder Ausrüstung der bioziden Filterschicht können Verbindungen auf der Basis von Poly(4-vinyl-N-hexylpyridiniumbromid) (Hexyl-PVP) verwendet werden. Dabei können sowohl Hexyl-PVP selbst oder aber Derivate dieser Verbindung oder andere Stoffe, die die Zellwand der Mikroorganismen physikalisch/mechanisch zerstören, verwendet werden.

Diese Beschichtung bietet den Vorteil einer bakteriziden Ausrüstung von Innenraumfilterelementen bei gleichzeitiger Schonung der Fahrgäste im Gegensatz zu diffundierenden Stoffen, die in den Fahrgastraum eindringen.

Eine polymere Beschichtung mit Hexyl-PVP ist in der Lage, die meisten der herkömmlichen Bakterienarten, die schwerwiegende Infektionen verursachen, abzutöten. Die Beschichtung beruht auf einer nicht-chemischen Einwirkung, um die Keime abzutöten. Das Polymer trägt eine positive Ladung, die harmlos für Menschen ist. Die Ladung reicht aus, um die Zellwände der Bakterien und ihre inneren Membranen zu zerstören. Bis zu 99% gefährlicher Mikroben wie zum Beispiel Staphylokokken, E. coli etc. können so abgetötet werden.

Das Hexyl-PVP kann auf chemischem Wege auf die Filterelemente aufgebracht werden und ist somit nicht abwaschbar. Lediglich abgetötete Bakterien, die aus der Luft auf der beschichteten Oberfläche landen, müssen gelegentlich entfernt werden.

Außerdem sind Ausrüstungen auf der Basis von Nanosilber und enzymatisch bzw. chemisch wirkendender Stoffe möglich wie z.B. 2-Brom-2-nitropropane-1,3-diol, Isothiazolinverbindungen, Benzoesäure, Benzalkoniumhalogenide, wasserlösliche Coenzyme, öllösliche Coenzyme, Pflanzenextrakte, Antibiotika, biozide Metalle, aliphatische und aromatische Fettsäuren.

Aus dem erfindungsgemäßen Filtermedium lässt sich ein Filtermodul, insbesondere ein Partikel- und/oder Geruchsfilter, mit einem Filtergehäuse herstellen, bei dem das Filtermedium in das Filtergehäuse eingefügt ist.

## Patentansprüche

1. Filtermedium mit bakterizider Wirkung, insbesondere zur Filterung von Luft für den Innenraum von Kraftfahrzeugen, bestehend aus mindestens einer Filterschicht, in der Verunreinigungen zurückhaltbar sind, und einer dieser Filterschicht nachgeschalteten bakteriziden Filterschicht, **dadurch gekennzeichnet, dass** die bakterizide Filterschicht auf der Reinluftseite der mindestens einen Filterschicht angebracht und durch eine Abstandsschicht von der mindestens einen Filterschicht beabstandet ist.

2. Filtermedium nach Anspruch 1, **dadurch gekennzeichnet, dass** die bakterizide Filterschicht Poly(4-vinyl-N-hexylpyridiniumbromid) enthält.

3. Filtermedium nach Anspruch 1, **dadurch gekennzeichnet, dass** die bakterizide Filterschicht ein Derivat von Poly(4-vinyl-N-hexylpyridiniumbromid) enthält.

4. Filtermedium nach Anspruch 1, **dadurch gekennzeichnet, dass** die bakterizide Filterschicht Stoffe enthält, die die Zellwand von Mikroorganismen physikalisch und/oder mechanisch zerstören können.

5. Filtermedium nach Anspruch 1, **dadurch gekennzeichnet, dass** die bakterizide Filterschicht Stoffe auf der Basis von Nanosilber enthält.

6. Filtermedium nach Anspruch 1, **dadurch gekennzeichnet, dass** die bakterizide Filterschicht Stoffe enthält, die ausgewählt sind aus der Gruppe bestehend aus 2-Brom-2-nitropropane-1,3-diol, Isothiazolinverbindungen, Benzoesäure, Benzalkoniumhalogenide, wasserlösliche Coenzyme, öllösliche Coenzyme, Pflanzenextrakte, Antibiotika, biozide Metalle, aliphatische und aromatische Fettsäuren.

7. Filtermedium nach einem der vorstehenden Ansprüche, **dadurch gekennzeichnet, dass** die Abstandsschicht als bakterizide Schicht ausgebildet ist.

8. Filtermedium nach einem der vorstehenden Ansprüche, **dadurch gekennzeichnet, dass** die Abstandsschicht aus einem feinen Kunststoffgitter, einer Klebermatrix oder einem 3-D-Fasergestrick oder -gewirk besteht.

9. Filtermedium nach einem der vorstehenden Ansprüche, **dadurch gekennzeichnet, dass** es zusätzlich eine Absorptionsschicht enthält.

10. Filtermedium nach Anspruch 9, **dadurch gekennzeichnet, dass** die Absorptionsschicht als Abstandsschicht fungiert.

11. Filtermodul, insbesondere Partikel- und/oder Geruchsfilter, mit einem Filtergehäuse mit zumindest einem Filtermedium nach einem der Ansprüche 1 bis 10, welches in das Filtergehäuse eingefügt ist.
